## Europäisches Patentamt
## European Patent Office
## Office européen des brevets

(19)

(11) Publication number: **0 054 391**
**A2**

# (12) EUROPEAN PATENT APPLICATION

(21) Application number: **81305762.7**

(51) Int. Cl.³: **A 61 F 1/08**

(22) Date of filing: **07.12.81**

(30) Priority: **08.12.80 GB 8039332**

(43) Date of publication of application:
**23.06.82 Bulletin 82/25**

(84) Designated Contracting States:
**AT BE CH DE FR IT LI LU NL SE**

(71) Applicant: **ROBERT KELLIE & SON LIMITED**
**Rutherford Road Dryburgh Industrial Estate**
**Dundee DD2 3XF Scotland(GB)**

(72) Inventor: **Bell, Gordon Andrew**
**3 Ballindean Crescent**
**Dundee Scotland(GB)**

(72) Inventor: **Duthie, James Edward**
**29 Melfort Place**
**Dundee Scotland(GB)**

(74) Representative: **Cole, Paul Gilbert et al,**
**Hughes Clark Andrews & Byrne 63 Lincoln's Inn Fields**
**London WC2A 3JU(GB)**

(54) Improvements in artificial limbs and fittings therefor.

(57) An artificial leg includes a low-height knee unit (1) pivoted on bolt (3) within a shin unit (2). The top face of the knee unit is formed with a generally frustoconical socket (32) formed at its mouth with a concave spherical seat (30). First, second and third screws (31) at 120 degree angular spacings fit in threaded bores so as to be tightened against recessed surfaces (25) formed on a head (24) of a male member having a convex spherical surface (22) that mates with seat (30). So manipulation of screws (31) permits the male member to be moved relative to the socket within a permitted range of adjustment. The shin unit is vertically extended to accommodate within it a swing phase control unit (12, 13) connected between the knee unit at pivot (14) posterior of knee pivot (12) and the shin unit at pivot (15) located just above shin tube (18). A resilient buffer (6) terminates pivotal motion in extension between the knee unit (1) and the shin unit (2) and a lock pawl (8) pivoted at (9) within the shin unit may be released by an operating cable (40, 41). The lower end of the shin tube (18) may be attached to the foot by a clamp (not shown) having three adjustable screws like the screws (31) about a frustoconical socket and receiving a male clamping member formed with a convex spherical surface and a head having three recessed faces engaged by the screws.

./...

FIG.1

IMPROVEMENTS IN ARTIFICIAL LIMBS

AND FITTINGS THEREFOR

This invention relates to artificial lower limbs and is especially concerned with an endo-skeletal prosthesis including a particular form of low height knee unit and an alignment system which is light, simple and easy to work with.

One form of artificial leg at present in use is made as a hollow monocoque structure in aluminium or wood or various types of laminated plastics, with all loads being taken through the contoured external material of which the limb is made. Such a structure can be very light in weight, but is not always nor necessarily so. Some of the disadvantages of this construction are that it normally requires skilled personnel to manufacture and can take weeks or months to finally complete. Much of this time element may be due to the difficulty of alignment and adjustment of this type of construction. Also, knee units require to be made in different sizes to match patients' dimensions and, particularly in smaller sizes, there may be limitations to the devices or mechanisms that can be used within the knee units.

A second form of artificial leg at present in use is made as a central strut structure, similar in nature to the human bone skeleton, the external shape of the artificial leg comprising a shaped flexible plastic foam fairing. This type of structure is known as endo-skeletal, and generally can be made up into a usable functioning limb in a few hours from a kit of standard parts.

It is an object of the present invention to provide an adjustable coupling between different parts of an artificial limb (eg. stump socket-thigh pylon, thigh-knee unit or shin tube-foot) that can be used at all stages of limb fitting, to enable rapid alignment of the foot, knee or stump socket as the case may be in any necessary angular or axial or length dimension as may be required, with a minimum component inventory.

It is another object of the invention to provide an artificial limb of endo-skeletal form with a single knee unit which can accept a plurality of mechanisms such that the limb may be used for the majority of normal above knee amputations. A further object of the present invention is to achieve a low height profile, ie. the minimum possible distance as measured from the knee pivot to the top of the knee unit including the alignment device. A yet further object of the present invention is to provide an artificial limb in which one form of mechanism can rapidly be replaced by another, if required, to match the limb function to the patient's ability during walking training, so that this one limb can be used for the patient throughout his rehabilitation, and finally be fitted with such mechanisms and fairing as found to be required to suit the mature patient, to become the permanent limb.

In one aspect there is provided a coupling for an artificial leg which, broadly stated, comprises a generally circular male member having a head formed with first, second and third recessed surfaces disposed at equal angular intervals about the axis of the head and a body of larger diameter than the head having a smoothly curved outer surface, and a female member having a central bore into which the head of the male member fits and having first, second and third screws in threaded bores in said female member disposed at equal angular intervals and directed away from the entrance of the central bore so that the said screws may be tightened against the recessed surfaces to draw the curved outer surface of the body into engagement with a complementary seat at the mouth of the bore.

It will be understood that a three screw clamp is the preferred arrangement on account of manufacturing simplicity and practicality. But an arrangement using four adjustable screws and four recessed surfaces could in principle be used - what is significant is that the minimum number of adjustable clamping screws is three. In a preferred feature a slot in the male member is engaged by a peg in the female member to prevent relative rotation between the male and female members when the screws are slackened off from engagement with the recessed surfaces.

The coupling may be fitted at the knee and shin joint in which case the female portion is preferably a socket in the knee part of a knee and shin pivot unit of an artificial leg which socket comprises means defining a generally frustoconical void having a concave spherical ring seating at its upper end, three adjustment and locking screws carried in its annular wall which adjustment and locking screws are spaced radially apart at an angle of one hundred and twenty degrees and inclined inwards and downwards at an angle of about fifteen degrees. In one form of male member there may be a flat base formed with fixing holes for attachment to a socket for the thigh stump of an above-knee leg amputee with its head received in a female member in the form of the knee portion of a knee and shin pivot. In another form of coupling the shin pivot receives a male member in the form of a shaped lower end of a pylon whose upper end is secured to a socket intended to receive a relatively short thigh stump of an above-knee leg amputee. The male member may be formed with a convex spherical surface having an anti-rotation slot and a head having three flat surfaces undercut and inclined at fourteen degrees to the vertical axis.

For use to attach a foot to the lower end of the shin tube, the female member is formed with means defining a generally frustoconical void having a concave spherical ring seating at its lower end, three adjustment and locking screws carried in its annular wall which adjustment and locking screws are spaced radially apart at an angle of about one hundred and twenty degrees and inclined inwards and upwards at an angle of about fifteen degrees, and a cylindrical extension for clamping engagement with the lower end of a shin tube. The male member may be formed with a baseplate for attachment to the top face of an artificial foot, a body on top of the baseplate having a convex spherical surface formed with an anti-rotation slot, and a head having three flat surfaces undercut and inclined at about fourteen degrees to the vertical axis.

The coupling may advantageously be used in association with a novel type of knee and shin unit that forms a second aspect of the invention. Thus the invention provides an artificial lower limb wherein the shin unit is elongate to house the swing phase control means which is connected at its upper end to the knee unit at a pivot posterior of the knee bolt and at its lower end to the shin unit at a pivot located adjacent the shin tube.

An advantage of having both the lock pawl and its operating cable carried in the knee part is that the effective lower part of the operating cable does not become bent as the knee flexes and can run straight from the point where it enters the knee unit to the lock pawl.

It is desirable that the swing phase control unit should not be placed above the knee pivot but instead should be housed substantially wholly in the shin unit that is vertically extended to accommodate it and is connected to a correspondingly abbreviated shin tube. So preferably the shin unit is elongate to house the swing phase control means which is connected at its upper end to the knee unit at a pivot posterior of the knee bolt and at its lower end to the shin unit at a pivot located adjacent the shin tube. Preferably the vertical levels of the buffer, knee pivot and upper pivot for the swing phase control means substantially coincide when the knee is in extension.

Preferably the lock pawl is pivotally carried within the knee unit anterior of the knee pivot and is spring loaded forwardly into engagement with an adjustable lock screw mounted in the shin unit, thereby locking the knee and shin parts in extension, a bowden cable arranged to rotate the lock pawl against spring tension when pulled to disengage said pawl from the screw. According to a further preferred feature the operating cable exits from the rear of the knee part so that it may curve round and be fitted on either a left or right socket.

An embodiment of the invention will now be described with reference to the accompanying drawings in which:

Figure 1 is a side view, part sectioned, of a knee and shin unit in accordance with the invention;

Figure 2 is a side view, part sectioned, of a three-screw clamp mounted on a foot coupling;

Figure 3 is a section on line x-x of Figure 2 showing the disposition of the alignment clamp and coupling parts in plan; and

Figures 4, 4A and 5 show views of the alignment couplings which can be fitted to the knee unit shown in Figure 1.

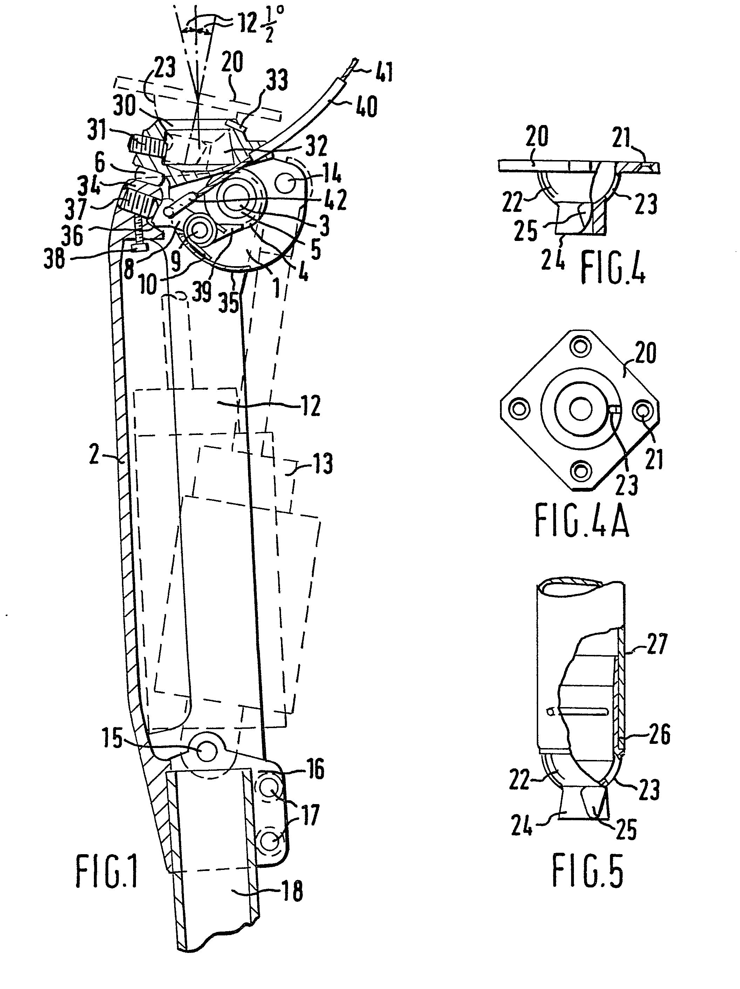

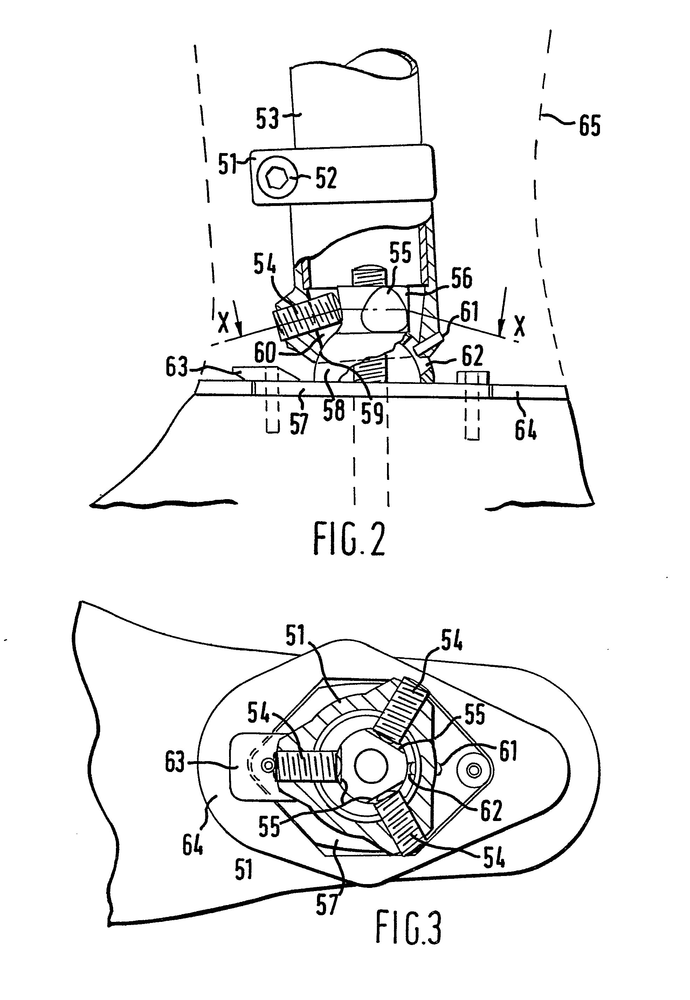

Referring to Figure 1, knee unit 1 is mounted between the side members of shin unit 2 by means of knee bolt 3 which passes through a pair of high load capacity ballraces 4 fitted into the knee unit 1. The ballraces are spaced apart by spacer 5. The knee unit is formed with a concave seating 30 for alignment couplings shown in Figures 4, 4A and 5; a screw 31 which is one of three arranged at equal angular spacings (as in Figure 3) around an annular void 32 whose inclined sidewall limits the angle of tilt of the alignment couplings to be placed therein, and a peg 33 which is set in the said concave seat. The purpose of peg 33 and the three screws 31 is fully described below with reference to Figure 2. The said alignment couplings may be angularly adjusted in any direction over an angle of twenty five degrees and locked in any preferred setting.

Extension bumpers 6 are located in shinstop 34 and when stop surface 35 impacts upon said bumpers, further rotation of the knee unit is prevented. The extension bumpers contain elements of relatively soft rubber and elements of relatively hard rubber so as to take up the impact in two stages. Lock pawl 8 is pivotally mounted within the knee unit at 9 and urged in an anti-clockwise direction by spring 10 such that it lies upon a ledge 36 and abuts the adjustable lock stop screw 37. Security screw 38 prevents loosening of the chosen setting of lock stop screw 37. The pawl tail 39 is designed to provide a limit stop to the motion of the pawl in the anti-clockwise direction where the tail would otherwise stop against spacer 5. When the knee is unlocked by pulling on cable 41 running in sheath 40, cable end 42 which is pivotally engaged in pawl 8 pulls the pawl in a clockwise direction until motion is stopped by the pawl tail 39 against stop 43.

Swing phase control of the knee is achieved by mechanical, hydraulic or pneumatic dampers of a known kind, outlined at 12 and 13, or other spring return devices (not shown). The dampers are mounted below the knee bolt 3 on pivots at 14 and 15 and during walking they control the angular velocity of the knee unit relative to the shin unit. The lower end of the shin unit 2 comprises a circular clamp 16 tightened by screws 17 upon the upper end of shin tube 18.

Referring to Figure 2, the lower shin tube clamp 51 has at its upper end a circular tube clamp which may be tightened by screw 52 upon the pylon tubing 53. At the lower end of clamp 51 are three screws 54 arranged equally around the diameter and inclined at an angle of $15^{\circ}$ such that together they exert an upward thrust upon the inclined flats 55 shaped into the head 56 of the foot coupling 57, pulling the convex spherical surface 58 into intimate fitting contact with the concave seating 59. The seating 59, shaped void 60 and screws 54 are identical respectively to the corresponding parts 30, 32 and 31 in Figure 1.

A feature is the peg 61 projecting inwards from the seating 59 at the rear of the tri-screw clamp. This peg runs in a vertical slot 62 cut in the convex surface 58, the purpose being to prevent relative rotation of part 51 around part 57 at times when the screws 54 are slackened off. This feature has been found to be of great benefit during fitting trials upon the patient and it is also present in the seating 30 of the knee unit shown in Figure 1. Another significant feature is the tab 63 which holds down foam attachment plate 64, this plate being required to locate and terminate the lower end of a foam fairing 65 (shown dotted).

Referring to Figure 3, the tri-screw alignment clamp 51 is shown with the three screws 54 spaced equally in a radial fashion around the diameter. These screws abut on the flats 55 of the coupling head 56, and adjustably locate the head and lock it in a selected position which thus holds lower shin tube or pylon clamp 51 at a required angle relatively to foot coupling 57. Also shown is peg° 61 locating in the slot 62, and tab 63 holding down plate 64 which is itself located round the base of foot coupling 57.

Figures 4 and 4A show the socket coupling used in the embodiment of Figure 1 having a square baseplate 20 and four stump socket attachment holes 21. Beneath baseplate 20 is an integrally formed convex spherical surface 22 corresponding to surface 58 in Figures 2 and 3 and slotted at 23 for angular location on peg 33. Beneath surface 22 is a head 24 corresponding to head 56 in Figures 2 and 3 and formed with flats 25 which are engaged by adjustable clamping screws 31. Figure 5 shows a pylon coupling which at its lower end has the same feature of three inclined flats 25 on a head 24 beneath a convex surface 22 that mates with seat 30 and is formed with anti-rotation slot 23 for peg 33 as already described in relation to Figures 2 and 3. But instead of baseplate 21 there is a tubular extension 26 that fits telescopically within a pylon tube 27 of appropriate length to extend the length of the prosthesis above the knee. A three-screw socket similar to that in Figures 2 and 3 may be provided at the top end of the pylon tube 27 for attachment of a coupling member attached to the stump socket.

CLAIMS:

1.    A coupling for  an artificial leg comprising a generally circular male member (57) having a head (56) formed with first, second and third recessed surfaces (55) disposed at equal angular intervals about the axis of the head and a body of larger diameter than the head and having a smoothly curved outer surface (58) and a female member (51) having a central bore 60 into which the head (56) of the male member ·fits and having first, second and third screws (54) in threaded bores in said female member disposed at equal angular intervals and directed away from the entrance of the central bore so that the said screws (54) may be tightened against the recessed surfaces (55) to draw the curved outer surface (58) of the body into engagement with a complementary seat (59) at the mouth of the bore.

2.    A coupling according to claim 1, wherein a slot (62) in the male member is engaged by a peg (61) in the female member to prevent relative rotation between the male and female members when the screws 54 are slackened off from engagement with the recessed surfaces (55).

3.    A coupling according to claim 1 or 2, wherein the female member is a socket in the knee part (1) of a knee and shin pivot unit (1, 2) of an artificial leg which socket comprises means defining a generally frustoconical void (32) having a concave spherical ring seating 30 at its upper end, three adjustment and locking screws (31) carried in its annular wall which adjustment and locking screws (31) are spaced radially apart at an angle of one hundred and twenty degrees and inclined inwards at an angle of about fifteen degrees with respect to the axis of spherical seat (30).

4.    A coupling according to claim 3, wherein the male member has a flat base (20) formed with fixing holes (21) for attachment to a socket for the thigh stump of an above-knee leg amputee with its head (24) received in a female member in the form of the knee portion of a knee and shin pivot.

5.    A coupling according to claim 3, wherein a female member in the form of a socket in the knee portion of a knee and shin pivot receives a male member in the form of a shaped lower end (22, 24, 25) of a pylon (27) whose upper end is secured to a socket intended to receive a relatively short thigh stump of an above-knee leg amputee.

6. A coupling according to claim 4 or 5, wherein the male member is formed with a convex spherical surface (22) having an anti-rotation slot (23) and a head (24) having three flat surfaces (25) undercut and inclined at about fourteen degrees to the vertical axis.

7.    A coupling according to claim 1 or 2, wherein the female member is formed with means defining a generally frustoconical void (60) having a concave spherical ring seat (59) at its lower end, three adjustment and locking screws (54) carried in its annular wall which adjustment and locking screws are spaced radially apart at an angle of about one hundred and twenty degrees and inclined inwards and upwards at an angle of about fifteen degrees, and a cylindrical extension for clamping engagement (51, 52) with the lower end of a shin tube (53).

8.    A coupling according to claim 7, wherein the male member (57) is formed with a baseplate for attachment to the top face of an artificial foot, a body on top of the baseplate having a convex spherical surface (58) formed with an anti-rotation slot (62), and a-head (56) having three flat surfaces (55) undercut and inclined at about fourteen degrees to the vertical axis.

9.    A coupling according to claim 8, wherein the baseplate further comprises a tab (63) for location of a cosmesis attachment plate.

10.    An artificial lower limb of the endo-skeletal type including a low height knee unit (1) having a socket (30, 32) at its upper end arranged to receive a coupling member (22, 24) connected to a stump socket and pivoted on a knee pivot (3) within a shin unit (2) connected at its lower end to a shin tube (18), a resilient buffer (6) to terminate pivotal motion in extension between the knee unit and the shin unit, a lock pawl (8) pivotally mounted within the knee unit and arranged to engage with a stop (37) mounted in the shin unit, a cable release (40, 41) for the lock pawl mounted in the knee unit and operable to rotate the pawl (8) about its pivot (9) to disengage it from the stop (37), and swing phase control means (12, 13) connected between the knee unit (1) and the shin unit (2) and located so that substantially the whole of said swing phase control means is below the knee pivot (3).

11.    An artificial lower limb according to claim 10 wherein the shin unit (2) is elongate to house the swing phase control means (12, 13) which is connected at its upper end to the knee unit at a pivot (14) posterior of the knee bolt (3) and at its lower end to the shin unit (2) at a pivot (15) located adjacent shin tube (18).

12.    A limb system according to claim 10 or 11  wherein the lock pawl (8) is pivotally carried within the knee part (1) anterior of the knee pivot (3) and is spring loaded forwardly into locking engagement with an adjustable lock screw (37) mounted in the shin unit, thereby locking the knee and shin parts in extension, a bowden cable (40, 41) arranged to rotate the lock pawl (8) against spring tension when pulled to disengage said pawl (8) from the lock screw (37).

13.    A limb system according to claim 12, wherein the lock operating cable (40, 41) exits from the rear of the knee part (1) so that it may curve round and be fitted on either a left or right socket.

FIG.1

FIG.4

FIG.4A

FIG.5

FIG.2

FIG.3